# EUROPÄISCHE PATENTSCHRIFT

(11) **EP 0 693 274 B1**
(45) Veröffentlichungstag und Bekanntmachung des Hinweises auf die Patenterteilung: **17.03.1999**
(21) Anmeldenummer: 95107289.1
(22) Anmeldetag: 13.05.1995
(51) Int. Cl.: A61B 17/70, A61F 2/44

(54) **Implantat zum Einsetzen zwischen Wirbelkörper der Wirbelsäule als Platzhalter**
Implant for maintaining the normal spacing between two vertebrae
Implant pour le maintien de l'écartement normal entre deux vertèbres

(30) Priorität: 02.07.1994 DE 4423257
(43) Veröffentlichungstag der Anmeldung: 24.01.1996
(73) Patentinhaber: ULRICH, Heinrich, D-89077 Ulm/Donau (DE)
(72) Erfinder: Schönhöffer, Helmut, Dr., D-89077 Ulm (DE)
(74) Vertreter: Fay, Hermann, Dipl.-Phys. Dr.

(56) Entgegenhaltungen:
- EP-A- 0 567 424
- AT-B- 395 524
- DE-A- 3 435 771
- FR-A- 263 622
- FR-A- 2 636 227
- GB-A- 2 083 754
- US-A- 4 657 550

## Beschreibung

Implantat zum Einsetzen zwischen Wirbelkörper der Wirbelsäule als Platzhalter.

Die Erfindung betrifft ein Implantat zum Einsetzen zwischen Wirbelkörper der Wirbelsäule als Platzhalter für aus der Wirbelsäule entfernte Wirbel oder Wirbelteile, bestehend aus zwei endständigen, zur Anlage an den angrenzenden Wirbeln bestimmten Implantatteilen und einem dazwischen befindlichen, mittigen Implantatteil, das mit den endständigen Implantatteilen durch je ein Gewinde verbunden ist, wobei die beiden Gewinde in Längsrichtung der Wirbelsäule koaxial angeordnet sind und zueinander gegenläufigen Gewindesinn besitzen, so daß durch Verdrehen des mittigen Implantatteils die Länge des Implantats insgesamt veränderbar ist, alle drei Implantatteile als rohrförmige Hülsen mit zumindest im Bereich der Gewinde kreisförmigen Querschnitt ausgebildet und koaxial ineinander geschraubt sind, und wobei die Hülsenwände des mittigen Implantatteils von Aussparungen durchbrochen sind.

Implantate dieser Art sind aus EP-A-0 567 424 bekannt, bei denen aufgrund der Dreiteiligkeit des Implantats mit der Verbindung über Gewinde eine einfache und einfach während der Operation zu handhabende Distraktionsmöglichkeit besteht.

Aus US-PS 4 657 550 sind Implantate bekannt, die durch Verdrehen des mittigen Implantatteils eine Distraktion des das Implantat enthaltenden Wirbelsäulenbereichs ermöglichen. Bei dem bekannten Implantat sind die beiden endständigen Implantatteile jeweils zweiteilig ausgebildet und bestehen je aus einer am angrenzenden Wirbel zur Anlage kommenden, zum Wirbel hin mit Dornen besetzten Druckplatte und einem Gewindebolzen, der mit einem Mehrkantkopf verdrehungsfest um die Bolzenachse in eine entsprechende Aufnahme in der Druckplatte eingesteckt ist. Das mittige Implantatteil ist mit Gewindebohrungen für die Aufnahme der Gewindebolzen ausgebildet. Dieses bekannte Implantat hat den Nachteil, daß nach dem Einsetzen des Implantats die Knochenbildung und -regeneration zu wünschen übrig läßt. Aus der DE 34 35 771 A1 ist ein diesen Nachteil vermeidendes Implantat bekannt, das mit einer Vielzahl von Löchern versehen ist, jedoch ist ein solches Implantat als Platzhalter für einen Wirbel oder Wirbelteil ungeeignet.

In der EP 0 268 115 B1 ist ein als Platzhalter für einen Wirbel dienendes Implantat beschrieben, das als mantelförmiges Element mit Ausnehmungen in der Wandung ausgebildet ist, wobei der obere und untere Mantelrand wenigstens teilweise zackenförmig ausgebildet sind, um eine drehfeste Verbindung zwischen den zu verbindenden Wirbeln zu ergeben. Das Element ist aus einem mit den Ausnehmungen versehenen Blech ein- oder zweilagig gewickelt. Durch die Ausnehmungen kann gewünschtenfalls ein in das Mantelinnere einzubringendes Material, wie Knochenzement oder Knochenstücke, eingefüllt werden, wodurch ein schnellerer Gefäßanschluß mit dem Implantat erreicht wird. Nachteilig bei einem solchen Implantat ist, daß es keinerlei Distraktion der benachbarten Wirbel ermöglicht.

Der Erfindung liegt die Aufgabe zugrunde, ein Implantat der eingangs genannten Art so auszubilden, daß es für die Induktion der Knochenbildung und Stimulation der Knochen besonders gut geeignet ist und daher nach der Implantation besonders leicht und schnell einwachsen kann.

Diese Aufgabe wird nach der Erfindung bei einem Implantat mit den eingangs genannten Merkmalen dadurch gelöst, daß die endständigen Implantatteile das mittige Implantatteil außenseitig axial übergreifen, wobei die Gewinde jeweils als Außengewinde auf dem mittigen Implantateil und als Innengewinde in den endständigen Implantatteilen ausgebildet sind, und daß die Hülsenwände der endständigen Implantatteile von Aussparungen durchbrochen sind.

Die erfindungsgemäße Ausbildung des aus drei Hülsen bestehenden Implantats mit axial das mittige Implantatteil außenseitig übergreifenden endständigen Implanttteilen läßt im Implantatinneren einen durchgehenden Hohlraum großen Volumens entstehen, der durch die in den Hülsenwänden befindlichen Aussparungen hindurch überall mit der Implantataußenseite in Verbindung steht. Dies gilt unabhängig vom Distraktionsstatus auch für die endständigen Implantatteile. Zusammen mit der Möglichkeit, den Hohlraum mit Knochenzement oder patienteneigenen oder fremden Knochenspänen zu füllen, läßt dies das Implantat schnell und zuverlässig einwachsen. Dennoch bleibt die Distraktionsmöglichkeit des Implantats während der Operation voll erhalten. Die relative Zuordnung von endständigen und mittigen Implantatteilen hat weiterhin zur Folge, daß die Gewinde nach außen hin weitgehend durch die endständigen Implantatteile abgedeckt sind.

Um im implantierten Zustand die Implantatteile gegen gegenseitiges Verdrehen zu sichern, kann mindestens eines der endständigen Implantatteile im Gewindebereich eine radiale Gewindebohrung aufweisen, in der eine gegen das mittige Implantatteil verspannbare Klemmschraube geführt ist, die im verspannten Zustand beide Implantatteile gegeneinander fixiert. Um die Hülsenwandung im Bereich der Klemmschraube zu stabilisieren, empfiehlt es sich, daß das mit der Klemmschraube versehene Implantatteil am axial innen liegenden Hülsenrand einen Ringbund mit im Vergleich zum restlichen Hülsenteil größerer Wandstärke aufweist und die Gewindebohrung für die Klemmschraube im Ringbund angeordnet ist. Um eine ausreichend große Führungslänge der Klemmschraube in der Gewindebohrung zu erhalten, ist zweckmäßigerweise der Ringbund im Bereich der Gewindebohrung mit einem radial auswärts vorstehenden Vorsprung versehen.

Vorzugsweise besitzt das mittige Implantatteil zwischen seinen beiden Gewinden einen gewindefreien Hülsenteil, in dem die Aussparungen gleichmäßig über den Hülsenumfang verteilt und als Schlüsselöffnungen zum Einstecken eines zum Verdrehen des mittigen Implantatteils dienenden Schlüssels ausgebildet sind.

Bezüglich der Ausbildung und Anordnung der Aussparungen außerhalb des gewindefreien Hülsenteils des mittigen Implantatteils besteht im Rahmen der Erfindung eine gewisse Wahlfreiheit. Einerseits soll der von den Aussparungen insgesamt repräsentierte lichte Querschnitt möglichst groß sein, andererseits dürfen die Aussparungen die Gewindebereiche nicht unzulässig schwächen, damit die Festigkeit der Gewindeverbindung zwischen den Implantatteilen keine Beeinträchtigung erfährt. Eine alle diese Forderungen besonders vorteilhaft erfüllende und daher im Rahmen der Erfindung bevorzugte Ausführungsform ist dadurch gekennzeichnet, daß die Aussparungen in den Implantatteilen im Bereich ihrer Gewinde vorgesehen und als Langlöcher mit in der Hülsenumfangsrichtung orientierter großer Lochachse ausgebildet sind. Zweckmäßigerweise sind die Aussparungen in Richtung ihrer großen Lochachse in einer zur Hülsenachse senkrechten Ebene gereiht. Zumeist wird man je Gewindebereich mehr als nur eine Lochreihe vorsehen. Dann sind bei mehreren Lochreihen im selben Gewindebereich die Aussparungen benachbarter Reihen in Umfangsrichtung zweckmäßigerweise gegeneinander versetzt. Bei derartig versetzten Aussparungen liegen die Aussparungen der einen Reihe mit ihrer Aussparungsmitte vorzugsweise in der Mitte zwischen jeweils zwei aufeinander folgenden Aussparungen der benachbarten Reihe. Im übrigen empfiehlt es sich, die Anordnung so zu treffen, daß die große Lochachse der Aussparungen länger, insbes. mehr als doppelt so lang wie der Randabstand zwischen den in jeweils der Reihe aufeinander folgenden Aussparungen ist, und daß der axiale Randabstand zwischen den in benachbarten Reihen angeordneten Aussparungen kleiner, insbes. etwa halb so groß wie die Länge der kleinen Lochachse ist. Bei derartigen Aussparungsanordnungen bleiben zwischen den benachbarten Aussparungen genügend große und in sich zusammenhängende Gewindebereiche bestehen, die die Lastübertragung zwischen den Implantatteilen zuverlässig gewährleisten.

Im allgemeinen ist die Kraftübertragung zwischen dem Implantat und dem angrenzenden Wirbel allein über den dem Wirbel anliegenden Hülsenrand der endständigen Implantatteile möglich. Sollte aber dabei die spezifische Kraftbeanspruchung am Wirbel zu groß werden, besteht die Möglichkeit, den Hülsenrand zu verbreitern oder überhaupt mit einer Querplatte zu überdecken, die dann wiederum von Aussparungen möglichst durchbrochen sein sollte. Im übrigen wird der Hülsenrand für die Wirbelanlage in im wesentlichen einer Ebene verlaufen, die je nach den Erfordernissen im Einzelfall etwa senkrecht oder auch geneigt zur Hülsenachse gerichtet sein kann. Selbstverständlich ist es aber auch möglich, daß der Hülsenrand in einer von einer Ebene abweichenden Fläche verläuft.

Weiter besteht im Rahmen der Erfindung die Möglichkeit, die endständigen Implantatteile an den angrenzenden Wirbeln dadurch gegen ungewünschte Verlagerungen zu sichern, daß der axial äußere Rand mindestens eines der endständigen Implantatteile mit einer oder mehreren, zum Eindringen in den angrenzenden Wirbel bestimmten Schneiden oder Spitzen versehen ist. Im einzelnen sind verschiedene Ausbildungen möglich. So kann die Schneide in Umfangsrichtung längs des Randes durchgehend oder von Lücken unterbrochen verlaufen. Die Schneiden können aber auch radial als Enden von längs des Randes aufeinander folgenden Vorsprüngen ausgebildet sein. Schließlich können die Spitzen von längs des Randes angeordneten, axial ausgerichteten Dornen gebildet sein. Um zu verhindern, daß die Schneiden oder Spitzen zu tief in die angrenzenden Wirbel eindringen, kann es sich empfehlen, daß am Implantatteil ein die Eindringtiefe der Schneiden oder Spitzen begrenzender, radial nach innen oder außen vorspringender Ringsteg vorgesehen ist, der als Anschlag für den angrenzenden Wirbelkörper dienen kann. Die radial äußere Randfläche dieses Ringstegs kann mit etwa axial gerichteten, konvexen Kanten versehen sein, die zum sicheren Sitz des Implantats beitragen.

Im folgenden wird die Erfindung an in der Zeichnung dargestellten Ausführungsbeispielen näher erläutert; es zeigen:
- Fig. 1: ein Implantat nach der Erfindung in einer Seitenansicht,
- Fig. 2: das mittige Implantatteil des Implantats nach Fig. 1 in einer Seitenansicht,

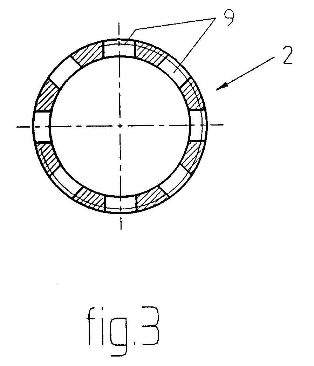

- Fig. 3: den Schnitt III - III in Fig. 2,
- Fig. 4: eine Seitenansicht eines endständigen Implantatteils in einer Ausführungsform mit im Vergleich zu dem Implantat nach Fig. 1 vergrößertem Durchmesser,
- Fig. 5: den Schnitt V - V in Fig. 4,
- Fig. 6: eine andere Ausführungsform eines endständigen Implantatteils nach Fig. 4,
- Fig. 7: den Schnitt VII - VII in Fig. 6,
- Fig. 8: den Schnitt VIII - VIII in Fig. 6,
- Fig. 9: den Schnitt IX - IX in Fig. 6,
- Fig. 10: eine nochmals andere Ausführungsform eines endständigen Implantatteils des erfindungsgemäßen Implantats in einer den Fig. 4 und 6 entsprechenden Darstellung,
- Fig. 11: den Schnitt XI - XI in Fig. 10, und
- Fig. 12: den Schnitt XII - XII in Fig. 11.

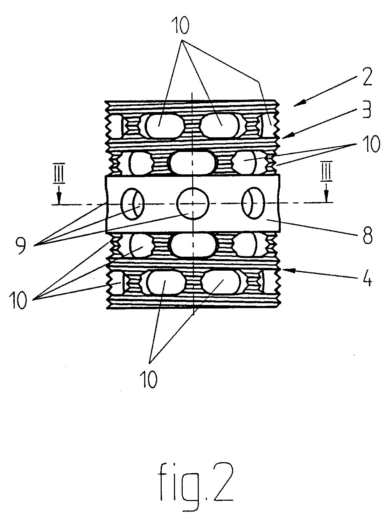

Das in der Zeichnung dargestellte Implantat dient zum Einsetzen zwischen in der Zeichnung selbst nicht dargestellte Wirbelkörper der Wirbelsäule als Platzhalter für aus der Wirbelsäule entfernte Wirbel oder Wirbelteile. Das Implantat besteht aus zwei endständigen, zur Anlage an den angrenzenden Wirbeln bestimmten Implantatteilen 1 und einem dazwischen befindlichen, mittigen Implantatteil 2. Dieses ist mit den endständigen Implantatteilen durch je ein Gewinde 3, 4 verbunden, wobei die beiden Gewinde in Längsrichtung der Wirbelsäule koaxial angeordnet sind und zueinander gegenläufigen Gewindesinn besitzen, wie dies insbes. aus Fig. 2 ersichtlich ist. Dadurch wird erreicht, daß beim Verdrehen des mittigen Implantatteils 2 relativ zu den beiden endständigen Implantatteilen 1 die Länge des Implantats insgesamt geändert und dabei insbes. der mit dem Implantat versehene Wirbelsäulenbereich bei der Implantation distrahiert werden kann.

Die endständigen Implantatteile 1 übergreifen in axialer Richtung das mittige Implantatteil 2 auf dessen Außenseite, wobei die Gewinde 3, 4 jeweils als Außengewinde auf dem mittigen Implantatteil 2 und als Innengewinde in den endständigen Implantatteilen 1 ausgebildet sind. Beide endständigen Implantatteile 1 besitzen im übrigen im Gewindebereich eine radiale Gewindebohrung 5, in der eine gegen das mittige Implantatteil verspannbare, in der Zeichnung der besseren Übersicht wegen selbst nicht dargestellte Klemmschraube geführt ist. Durch Verspannen dieser Klemmschraube jeweils gegen das mittige Implantatteil 2 werden die Implantatteile 1, 2 gegen gegenseitiges Verdrehen fixiert. Dabei können die endständigen Implantatteile 1 am jeweils axial innen liegenden Hülsenrand einen Ringbund 6 mit im Vergleich zum restlichen Hülsenteil größerer Wandstärke aufweisen. Die Gewindebohrung 5 für die Klemmschraube ist dann in diesem Ringbund 6 angeordnet, der das endständige Implantatteil gegen die bei der Verspannung der Klemmschraube auftretenden Kräfte stabilisiert. Auch kann der Ringbund 6 im Bereich der Gewindebohrung 5 einen radial auswärts vorstehenden Vorsprung 7 erhalten, so daß in der Gewindebohrung 5 eine ausreichend große Führungslänge für die Klemmschraube zur Verfügung steht.

Das mittige Implantatteil 2 besitzt zwischen seinen beiden Gewinden 3, 4 einen gewindefreien Hülsenteil 8. In diesem gewindefreien Hülsenteil 8 sind Aussparungen 9 gleichmäßig über den Hülsenumfang verteilt und als Schlüsselöffnungen zum Einstecken eines nicht dargestellten, zum Verdrehen des mittigen Implantatteils 2 dienenden Schlüssels ausgebildet. Im Ausführungsbeispiel haben diese Schlüsselöffnungen die Form kreiszylindrischer Löcher, so daß der Schlüssel in besonders einfacher Ausführungsform lediglich von einem am Einsteckende kreiszylindrischen Stab gebildet sein kann.

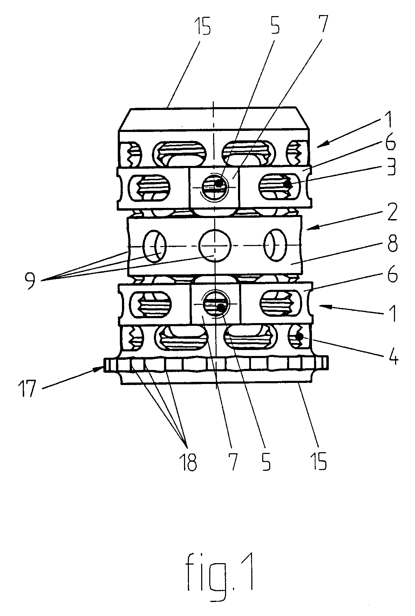

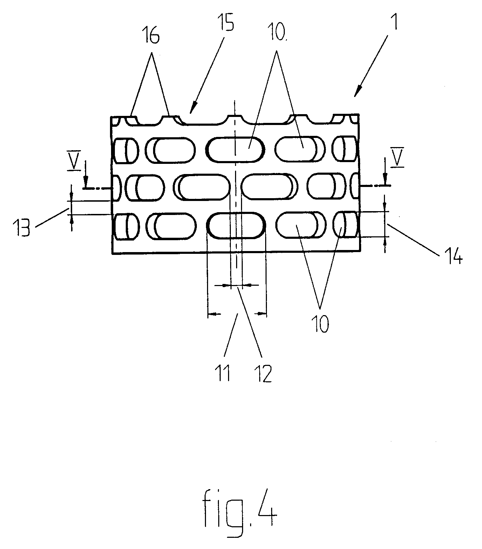

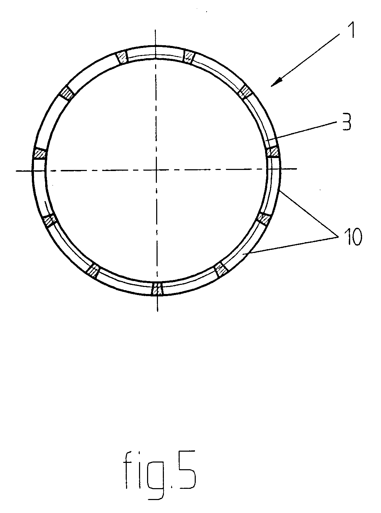

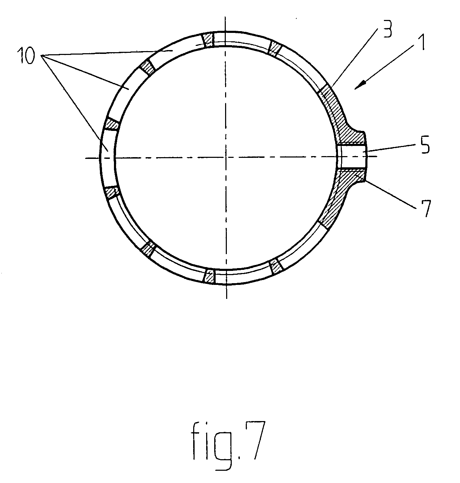

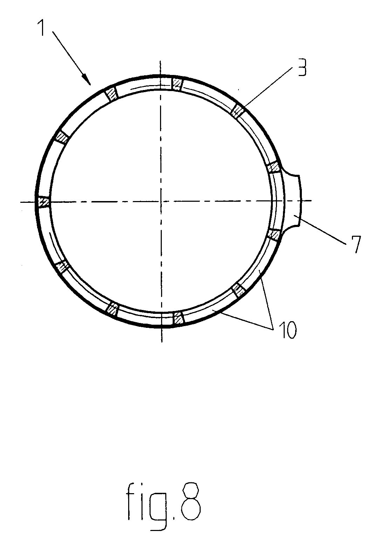

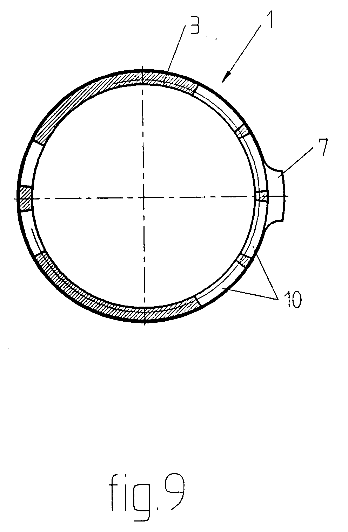

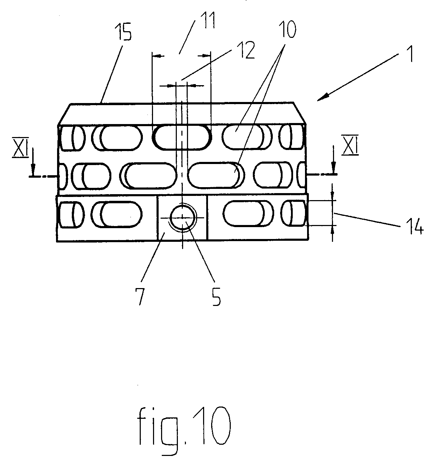

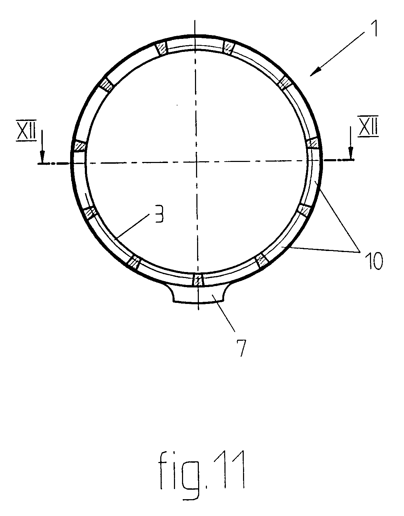

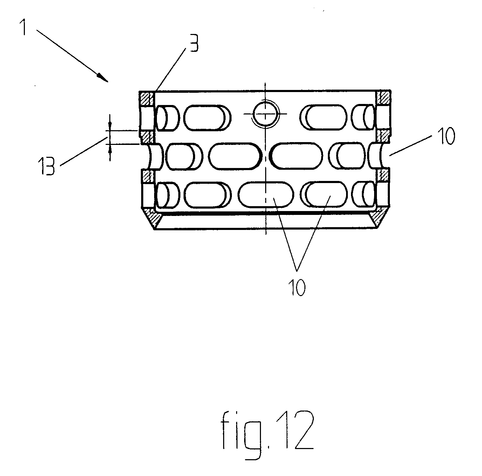

Alle anderen Aussparungen 10 in den drei Implantatteilen 1, 2 sind im Bereich von deren Gewinde 3, 4 vorgesehen und als Langlöcher mit in der Hülsenumfangsrichtung orientierter großer Lochachse 11 ausgebildet. Die Aussparungen 10 sind in Richtung ihrer großen Lochachse 11 in einer zur Hülsenachse senkrechten Ebene gereiht. Im Ausführungsbeispiel nach den Fig. 1 und 2 sind im jeweils selben Gewindebereich 3, 4 zwei solcher Lochreihen vorhanden, während in den Ausführungsbeispielen nach den Fig. 4 bis 12 wenigstens drei Lochreihen vorgesehen sind. Dabei sind die Aussparungen 10 benachbarter Reihen in Umfangsrichtung gegeneinander versetzt, wobei im einzelnen die Anordnung so getroffen ist, daß die Aussparungen jeweils einer der Reihen mit ihrer Aussparungsmitte in der Mitte zwischen jeweils zwei aufeinander folgenden Aussparungen der benachbarten Reihe bzw. Reihen liegen. Die große Lochachse 11 der Aussparungen 10 ist mehr als doppelt so lang wie der Randabstand 12 zwischen den in jeweils der Reihe aufeinander folgenden Aussparungen. Der axiale Randabstand 13 zwischen den in benachbarten Reihen angeordneten Aussparungen ist etwa halb so groß wie die Länge der kleinen Lochachse 14.

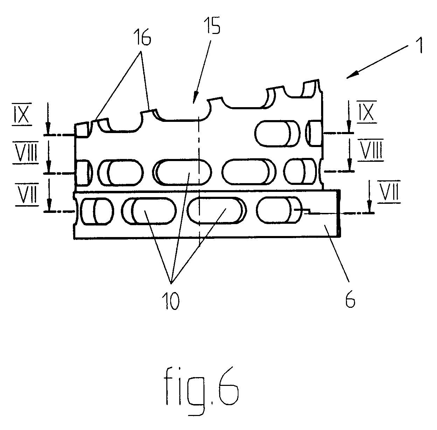

Im Ausführungsbeispiel nach den Fig. 1, 4 und 10 verläuft der axial äußere, zur Anlage am angrenzenden Wirbel bestimmte Rand 15 des endständigen Implantatteils 1 in einer zur Hülsenachse senkrechten Ebene. Im Ausführungsbeispiel nach Fig. 6 dagegen verläuft dieser Rand 15 in einer gegen die Hülsenachse geneigten Ebene, wobei im Ausführungsbeispiel der Neigungswinkel 10° beträgt. Dieser axial äußere Rand 15 kann mit einer Schneide, wie in den Ausführungsbeispielen nach den Fig. 1, 10 und 12, oder, wie in den Fig. 4 und 6, mit längs des Randes 15 aufeinander folgenden Vorsprüngen 16 versehen sein, wobei die Schneiden bzw. Vorsprünge den Zweck haben, die endständigen Implantatteile 1 in ihrer Anlage an den angrenzenden Wirbeln gegen seitliche Versetzungen oder Verlagerungen zu sichern, indem die Schneide bzw. Vorsprünge mehr oder weniger tief in den angrenzenden Wirbel eindringen. Auch die Vorsprünge 16 können ihrerseits mit Schneiden versehen sein, die in Umfangsrichtung oder quer dazu orientiert sind, um das Eindringen der Vorsprünge in den Wirbel zu erleichtern. Um dabei die Eindringtiefe zu begrenzen, kann, wie im Ausführungsbeispiel nach Fig. 1, am unteren endständigen Implantatteil 1 ein radial nach außen vorspringender Ringsteg 17 vorgesehen sein, der als Anschlag für den angrenzenden Wirbel dient. Die äußere Randfläche des Ringstegs 17 ist mit axial gerichteten, nach außen vorstehenden, d. h. konvexen Kanten 18 versehen.

## Patentansprüche

1. Implantat zum Einsetzen zwischen Wirbelkörper der Wirbelsäule als Platzhalter für aus der Wirbelsäule entfernte Wirbel oder Wirbelteile, bestehend aus zwei endständigen, zur Anlage an den angrenzenden Wirbeln bestimmten Implantatteilen (1) und einem dazwischen befindlichen, mittigen Implantatteil (2), das mit den endständigen Implantatteilen (1) durch je ein Gewinde (3, 4) verbunden ist, wobei die beiden Gewinde (3, 4) in Längsrichtung der Wirbelsäule koaxial angeordnet sind und zueinander gegenläufigen Gewindesinn besitzen, so daß durch Verdrehen des mittigen Implantatteils (2) die Länge des Implantats insgesamt veränderbar ist, alle drei Implantatteile (1, 2) als rohrförmige Hülsen mit zumindest im Bereich der Gewinde (3, 4) kreisförmigem Querschnitt ausgebildet und koaxial ineinander geschraubt sind, und wobei die Hülsenwände des mittigen Implantatteils (2) von Aussparungen (9, 10) durchbrochen sind, dadurch gekennzeichnet, daß die endständigen Implantatteile (1) das mittige Implantatteil (2) außenseitig axial übergreifen, wobei die Gewinde (3, 4) jeweils als Außengewinde auf dem mittigen Implantatteil (2) und als Innengewinde in den endständigen Implantatteilen (1) ausgebildet sind, und daß die Hülsenwände der endständigen Implantatteile (1) von Aussparungen (10) durchbrochen sind.

2. Implantat nach Anspruch 1, dadurch gekennzeichnet, daß mindestens eines der endständigen Implantatteile (1) im Gewindebereich (3, 4) eine radiale Gewindebohrung (5) aufweist, in der eine gegen das mittige Implantatteil (2) verspannbare Klemmschraube geführt ist.

3. Implantat nach Anspruch 2, dadurch gekennzeichnet, daß das mit der Klemmschraube versehene Implantatteil (1) am axial innen liegenden Hülsenrand einen Ringbund (6) mit im Vergleich zum restlichen Hülsenteil größerer Wandstärke aufweist und die Gewindebohrung (5) für die Klemmschraube im Ringbund (6) angeordnet ist.

4. Implantat nach Anspruch 3, dadurch gekennzeichnet, daß der Ringbund (6) im Bereich der Gewindebohrung (5) einen radial auswärts vorstehenden Vorsprung (7) aufweist.

5. Implantat nach einem der Ansprüche 1 bis 4, dadurch gekennzeichnet, daß das mittige Implantatteil (2) zwischen seinen beiden Gewinden (3, 4) einen gewindefreien Hülsenteil (8) aufweist, in dem die Aussparungen (9) gleichmäßig über den Hülsenumfang verteilt und als Schlüsselöffnungen zum Einstecken eines zum Verdrehen des mittigen Implantatteils (2) dienenden Schlüssels ausgebildet sind.

6. Implantat nach einem der Ansprüche 1 bis 5, dadurch gekennzeichnet, daß die Aussparungen (10) in den Implantatteilen (1, 2) im Bereich ihrer Gewinde (3, 4) vorgesehen und als Langlöcher mit in der Hülsenumfangsrichtung orientierter großer Lochachse (11) ausgebildet sind.

7. Implantat nach Anspruch 6, dadurch gekennzeichnet, daß die Aussparungen (10) in Richtung ihrer großen Lochachse (11) in einer zur Hülsenachse senkrechten Ebene gereiht sind.

8. Implantat nach Anspruch 7, dadurch gekennzeichnet, daß bei mehreren Lochreihen im selben Gewindebereich (3, 4) die Aussparungen (10) benachbarter Reihen in Umfangsrichtung gegeneinander versetzt sind.

9. Implantat nach Anspruch 8, dadurch gekennzeichnet, daß bei versetzten Aussparungen (10) die der einen Reihe mit ihrer Aussparungsmitte in der Mitte zwischen jeweils zwei aufeinander folgenden Aussparungen der benachbarten Reihe liegen.

10. Implantat nach einem der Ansprüche 7 bis 9, dadurch gekennzeichnet, daß die große Lochachse (11) der Aussparungen (10) länger, insbes. mehr als doppelt so lang wie der Randabstand (12) zwischen den in jeweils der Reihe aufeinander folgenden Aussparungen ist.

11. Implantat nach einem der Ansprüche 7 bis 10, dadurch gekennzeichnet, daß der axiale Randabstand (13) zwischen den in benachbarten Reihen angeordneten Aussparungen (10) kleiner, insbes. etwa halb so groß wie die Länge der kleinen Lochachse (14) ist.

12. Implantat nach einem der Ansprüche 1 bis 11, dadurch gekennzeichnet, daß der axial äußere Rand (15) mindestens eines der endständigen Implantatteile (1) in einer gegen die Hülsenachse geneigten Ebene verläuft.

13. Implantat nach einem der Ansprüche 1 bis 12, dadurch gekennzeichnet, daß der axial äußere Rand (15) mindestens eines der endständigen Implantatteile (1) mit einer oder mehreren, zum Eindringen in den angrenzenden Wirbel bestimmten Schneiden oder Spitzen versehen ist.

14. Implantat nach Anspruch 13, dadurch gekennzeichnet, daß die Schneide in Umfangsrichtung längs des Randes (15) durchgehend oder von Lücken unterbrochen verläuft.

15. Implantat nach Anspruch 13, dadurch gekennzeichnet, daß die Schneiden radial als Enden von längs des Randes (15) aufeinander folgenden Vorsprüngen (16) ausgebildet sind.

16. Implantat nach Anspruch 13, dadurch gekennzeichnet, daß die Spitzen von längs des Randes (15) angeordneten axial ausgerichteten Dornen gebildet sind.

17. Implantat nach einem der Ansprüche 13 bis 16, dadurch gekennzeichnet, daß am endständigen Implantatteil (1) ein die Eindringtiefe der Schneiden oder Spitzen begrenzender, radial nach innen oder außen vorspringender Ringsteg (17) vorgesehen ist.

## Claims

1. An implant for insertion between vertebral bodies of the spinal column as spacers for vertebrae or vertebra portions which are removed from the spinal column, comprising two terminal implant portions (1) intended to bear against the adjoining vertebrae and a central implant portion (2) which is disposed between the terminal implant portions and which is connected to the terminal implant portions (1) by a respective screwthread (3, 4), wherein the two screwthreads (3, 4) are arranged coaxially in the longitudinal direction of the spinal column and have mutually opposite screwthread directions so that the length of the implant overall is variable by rotating the central implant portion (2), all three implant portions (1, 2) are in the form of tubular sleeves of circular cross-section at least in the region of the screwthreads (3, 4) and are coaxially screwed one into the other, and wherein the sleeve walls of the central implant portion (2) are apertured by openings (9, 10), characterised in that the terminal implant portions (1) axially engage over the central implant portion (2) at the outside, wherein the screwthreads (3, 4) are respectively in the form of a male screwthread on the central implant portion (2) and a female screwthread in the terminal implant portions (1), and that the sleeve walls of the terminal implant portions (1) are apertured by openings (10).

2. An implant according to claim 1 characterised in that at least one of the terminal implant portions (1) has in the screwthread region (3, 4) a radial screwthreaded bore (5) in which is guided a clamping screw which can be tightened against the central implant portion (2).

3. An implant according to claim 2 characterised in that the implant portion (1) provided with the clamping screw has at the axially inwardly disposed sleeve edge an annular collar (6) of a wall thickness which is larger in comparison with the remaining part of the sleeve and the screwthreaded bore (5) for the clamping screw is arranged in the annular collar (6).

4. An implant according to claim 3 characterised in that the annular collar (6) has in the region of the screwthreaded bore (5) a radially outwardly protruding projection (7).

5. An implant according to one of claims 1 to 4 characterised in that between its two screwthreads (3, 4) the central implant portion (2) has a thread-free sleeve portion (8) in which the openings (9 ) are distributed uniformly around the periphery of the sleeve and are in the form of wrench openings for the insertion of a wrench serving to rotate the central implant portion (2).

6. An implant according to one of claims 1 to 5 characterised in that the openings (10) in the implant portions (1, 2) are provided in the region of their screwthreads (3, 4) and are in the form of elongate holes with their long hole axis (11) oriented in the peripheral direction of the sleeve.

7. An implant according to claim 6 characterised in that the openings (10) are arranged in a row in the direction of their long hole axis (11) in a plane perpendicular to the sleeve axis.

8. An implant according to claim 7 characterised in that when there are a plurality of rows of holes in the same screwthread region (3, 4) the openings (10) of adjacent rows are arranged in mutually displaced relationship in the peripheral direction.

9. An implant according to claim 8 characterised in that in the case of displaced openings (10) those of the one row are disposed with the centre of their openings in the centre between each two successive openings of the adjacent row.

10. An implant according to one of claims 7 to 9 characterised in that the long hole axis (11) of the openings (10) is longer than and in particular is more than twice as long as the edge spacing (12) between the openings which occur in succession in the respective row.

11. An implant according to one of claims 7 to 10 characterised in that the axial edge spacing (13) between the openings (10) arranged in adjacent rows is less than and in particular is approximately half as great as the length of the short hole axis (14).

12. An implant according to one of claims 1 to 11 characterised in that the axially outer edge (15) of at least one of the terminal implant portions (1) extends in a plane which is inclined with respect to the sleeve axis.

13. An implant according to one of claims 1 to 12 characterised in that the axially outer edge (15) of at least one of the terminal implant portions (1) is provided with one or more cutting edges or points intended to penetrate into the adjoining vertebra.

14. An implant according to claim 13 characterised in that the cutting edge is continuous or extends interrupted by gaps in the peripheral direction along the edge (15).

15. An implant according to claim 13 characterised in that the cutting edges are radially in the form of ends of projections (16) which occur in succession along the edge (15).

16. An implant according to claim 13 characterised in that the points are formed by axially directed spikes arranged along the edge (15).

17. An implant according to one of claims 13 to 16 characterised in that provided on the terminal implant portion (1) is a radially inwardly or outwardly projecting annular flange (17) which delimits the depth of penetration of the cutting edges or points.

## Revendications

1. Implant destiné à être placé entre des éléments de la colonne vertébrale en tant que moyen de remplacement pour des vertèbres ou des parties de vertèbres retirées de la colonne vertébrale, comprenant deux éléments d'implant d'extrémité (1) destinés à venir en appui sur les vertèbres voisines et un élément d'implant central (2) disposé entre deux, qui sont reliés chaque fois par un filetage (3, 4), les deux filetages (3, 4) étant disposés coaxialement dans la direction longitudinale de la colonne vertébrale et présentant des pas opposés de telle sorte qu'en tournant l'élément d'implant central (2) on puisse faire varier la longueur totale de l'implant, les trois éléments d'implant (1, 2) étant conformés en manchons tubulaires avec une section circulaire au moins dans la région des filetages (3,4) et étant vissés coaxialement l'un dans l'autre, les parois de l'élément d'implant central (2) étant pourvues de découpes (9, 10), caractérisé par le fait que les éléments d'implants d'extrémité (1) recouvrent extérieurement dans la direction axiale l'élément d'implant central (2), les filetages (3, 4) étant agencés respectivement sous forme de filetages extérieurs sur l'élément d'implant central (2) et sous forme de filetages intérieurs dans les éléments d'implant d'extrémité (1), et par le fait que les parois des éléments d'implant d'extrémité (1) sont pourvues de découpes (10).

2. Implant selon la revendication 1, caractérisé par le fait qu'au moins un des éléments d'implant d'extrémité (1), dans la zone filetée (3, 4), est pourvu d'un trou fileté (5) dans lequel est insérée une vis de blocage serrée sur l'élément d'implant central (2).

3. Implant selon la revendication 2, caractérisé par le fait que l'élément d'implant (1) pourvu de la vis de blocage présente au niveau de son bord situé axialement vers l'intérieur un collet annulaire (6) avec une épaisseur de paroi plus forte par rapport au reste de l'élément en forme de manchon et que le trou fileté (5) pour la vis de serrage est aménagé dans le collet annulaire (6).

4. Implant selon la revendication 3, caractérisé par le fait que le collet annulaire (6), dans la région du trou fileté (5), présente une saillie (7) qui s'étend radialement vers l'extérieur.

5. Implant selon une des revendications 1 à 4, caractérisé par le fait que l'élément d'implant central (2) présente, entre ses deux filetages (3, 4) une partie de manchon (8) non filetée dans laquelle les découpes sont uniformément réparties sur le pourtour du manchon et sont conformées en ouvertures de clé pour l'insertion d'une clé permettant de tourner l'élément d'implant central (2).

6. Implant selon une des revendications 1 à 5, caractérisé par le fait que les découpes (10) dans les éléments d'implant (1, 2) sont prévues dans la région des filetages (3, 4) desdits éléments et sont conformées en trous oblongs dont le grand axe (11) est orienté dans la direction périphérique du manchon.

7. Implant selon la revendication 6, caractérisé par le fait que les découpes (10), dans la direction de leur grand axe (11) sont alignées dans un plan perpendiculaire à l'axe du manchon.

8. Implant selon la revendication 7, caractérisé par le fait que lorsque plusieurs rangées de trous sont prévues dans la même zone filetée (3, 4) les découpes (10) de deux rangées consécutives sont mutuellement décalées dans la direction périphérique.

9. Implant selon la revendication 8, caractérisé par le fait que dans le cas de découpes (10) décalées, le milieu des découpes appartenant à une première rangée est situé au milieu entre chaque fois deux découpes consécutives de la rangée voisine.

10. Implant selon une des revendications 7 à 9, caractérisé par le fait que le grand axe (11) des découpes (10) est plus long, en particulier est deux fois plus long que la distance bord à bord (12) entre les découpes consécutives appartenant à la rangée considérée.

11. Implant selon une des revendications 7 à 10, caractérisé par le fait que la distance bord à bord (13) dans la direction axiale entre les découpes (10) appartenant à deux rangées consécutives est inférieure, en particulier est moitié moins grande que la longueur du petit axe (14) de découpe.

12. Implant selon une des revendications 1 à 11, caractérisé par le fait que le bord (15) situé axialement à l'extérieur d'au moins un des éléments l'implant d'extrémité (1) s'étend dans un plan incliné par rapport à l'axe du manchon.

13. Implant selon une des revendications 1 à 12, caractérisé par le fait que le bord (15) situé axialement vers l'extérieur d'au moins un des éléments d'implant d'extrémité (1) est pourvu d'une ou de plusieurs arêtes ou pointes destinées à pénétrer dans les vertèbres voisines.

14. Implant selon la revendication 13, caractérisé par le fait que l'arête, dans la direction périphérique, s'étend le long du bord (15), de manière continue ou est interrompue par des espaces.

15. Implant selon la revendication 13, caractérisé par le fait que les arêtes, dans la direction radiale, sont conformées en extrémités de saillies consécutives (16) disposées le long du bord (15).

16. Implant selon la revendication 13, caractérisé par le fait que les pointes sont formées par des pics dirigés axialement le long du bord (15).

17. Implant selon une des revendications 13 à 16, caractérisé par le fait qu'il est prévu sur l'élément d'implant d'extrémité (1) un cordon annulaire (17) qui fait saillie radialement vers l'intérieur ou vers l'extérieur et limite la profondeur de pénétration des arêtes ou des pointes.
